Europäisches Patentamt

European Patent Office

Office européen des brevets

(11) Publication number: **0 561 002 A1**

# EUROPEAN PATENT APPLICATION

(21) Application number: **91307097.5**

(22) Date of filing: **01.08.91**

(51) Int. Cl.5: **A61L 31/00, A61F 13/36**

(43) Date of publication of application:
**22.09.93 Bulletin 93/38**

(84) Designated Contracting States:
**DE FR GB IT SE**

(71) Applicant: **JAPAN GORE-TEX, INC.**
**42-5, 1-chome Akazutsumi**
**Setagaya-ku Tokyo 156(JP)**

(72) Inventor: **Nomi, Haruo**
**1-15-9, Sakauragaoka-Nishi, Sanyou-Cho**
**Akaiwa-gun, Okayama-Ken 709-08(JP)**
Inventor: **Hamasaki, Sadakatu**
**134-199 Minamikozu, Okayama-Shi**
**Okayama-Ken 709-08(JP)**
Inventor: **Takakiwa, Nobuo**
**3-60-2 Kami-Ishihara**
**Chofu-shi, Tokyo 182(JP)**

(74) Representative: **McCallum, William Potter et al**
**Cruikshank & Fairweather 19 Royal**
**Exchange Square**
**Glasgow G1 3AE Scotland (GB)**

(54) **A surgical pad.**

(57) A surgical pad comprised of a porous expanded fluorine resin sheet (1) having a porosity of 75 to 95% and a thickness of at least about 0.1 mm. The surgical pad can also be used as a cushioning material during surgery.

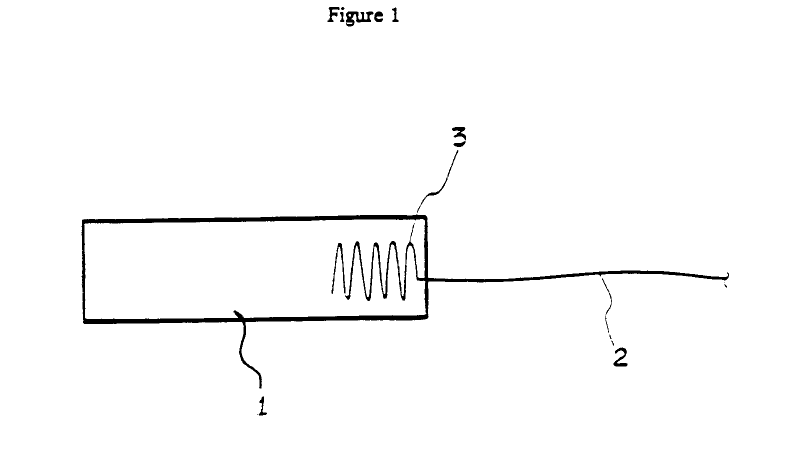

Figure 1

The present invention relates to a surgical pad that is used in surgical operations.

In surgical operations, surgical pads are used in order to protect internal organs and other tissue, to absorb blood, to ensure the field of vision, and for other objectives. Surgical pads that were used in the past were made from cotton, collagen, sea sponge, polyvinyl alcohol, acrylic resins, and other hydrophilic materials.

Surgical pads are particularly useful as cushioning materials during surgeries. In craniotomies (cranial nerve surgery), open-heart surgery (cardiac surgery), and peritoneotomies (abdominal surgery), surgical pads are used for cushioning in order to prevent the surgical equipment from putting undue pressure on tissue or coming in contact with tissue other than in the required places. In the past, woven and non-woven cloths made from cotton, collagen, polyvinyl alcohol, and other hydrophilic materials were used for these cushioning applications.

However, surgical pads made from such hydrophilic materials entailed various problems, including 1) they absorbed or adsorbed the blood and other body fluids generated during the operation and were dyed by these fluids, making it difficult to distinguish the surgical pad from the tissue, 2) the absorption of body fluids would result in a change of the properties of the surgical pad, such as swelling or contraction, 3) lint tended to be generated, which became a source of contamination, 4) the materials were hard to work with because they did not slide easily, and 5) when the operation extended over a long period of time, the coagulation of the blood absorbed by the surgical pad or the plating out of fibrin caused the surgical pad to adhere to the tissue, When the surgical pad was forcibly peeled away, a new contusion frequently resulted.

According to the present invention, there is provided a surgical pad comprising an expanded porous fluorine resin sheet having a porosity of 75 to 95% and a thickness of at least about 0.1 mm. Preferably, the fluorine resin is polytetrafluoroethylene

It is known that fluorine resins, which are used as the raw material for the surgical pad of the present invention, generally produce no sensitivity in living organisms and are anti-coagulants, and as such are used as artificial veins, blood accesses, pericardial patches, and other man-made tissue materials. The present invention relates to the use of these fluorine resins as materials for use in the manufacture of surgical pads used in surgical operations. Further, when these resins are made porous by stretching and then made into a sheet having a specific porosity and thickness, all of the drawbacks entailed in surgical pads made from conventional hydrophilic materials are eliminated, making this new material extremely useful as a surgical pad for use in surgical operations.

The surgical pad of the present invention is particularly useful as a cushioning material during surgeries.

An embodiment of the present invention will now be described, by way of example, with reference to the accompanying drawings in which:-

Fig. 1 is a diagrammatic representation of one form of the present invention.

Methods for manufacturing a porous expanded sheet from a fluorine resin have long been well known, examples of which include those discussed in Japanese Patent Publications 51-18991, 56-45773 and 56-17216 and US Patents 3953566 and 4187390. These materials have a microstructure of nodes interconnected by fine fibrils. The fibrils are oriented in the direction of stretching, that is, in the direction of expansion. Depending on the manufacturing conditions therein, the fibril length, porosity, and thickness can be arbitrarily varied. In addition to polytetrafluoroethylene (PTFE), examples of fluorine resins include copolymers of tetrafluoroethylene and hexafluoropropylene, and polyvinylidene fluoride. In the present invention, the use of PTFE is preferable.

The surgical pad of the present invention, which is composed of a porous expanded fluorine resin sheet, may consist of a single sheet or two or more sheets. If the material is to be used as a laminate, each sheet is integrally bonded with the others by means of thermal fusion or an adhesive agent. In this case, the porosity of the laminate is stipulated to be 75 to 95%, and the thickness to be greater than about 0.1 mm. Porosity is herein meant as the ratio of void volume to total volume expressed as a percentage of the density of non-porous PTFE, 2.2 g/cm$^3$.

In the surgical pad of the present invention, the porosity thereof is 75 to 95%, and preferably 80 to 90%. If the porosity is less than 75%, the shape conformability and end flexibility will be insufficient, making the material unsuitable as a surgical pad. On the other hand, if 95% is exceeded, the material will be difficult to work with (poor handling). The maximum micropore diameter of the surgical pad should be about 0.1 to 10 $\mu$m, and preferably about 0.2 to 3.0 $\mu$m. This is determined by ethanol bubble point measurement, where the maximum pore diameter in microns equals 0.65 divided by the ethanol bubble point value expressed in kg/cm$^2$.

In the surgical pad of the present invention, the thickness should be at least about 0. 1mm, and preferably about 0. 3 to 0. 7 mm. At a thickness of less than about 0.1 mm, the cushioning properties will be poor. If a thickness of 1 mm is exceeded, the handling will not be as good, although still acceptable for many applications.

Because the surgical pad of the present invention is flexible and has extremely low impact resilience, it can conform to small shapes, so that the tissue which has to be protected can be covered softly and closely without damaging it. Furthermore, because this surgical pad is very compressible, the material exhibits a superior effect as an energy absorber. Even if the edges of sheet come into contact with the tissue, the possibility of damaging the tissue is extremely slight. Moreover, because the material is water repellent, and does not absorb or adsorb body fluids, there will be no change in the color or properties of the sheet even during operations of extended periods of time. Consequently, the surgical pad of the present invention does not lose any of its energy absorbing action (cushioning action) even during operations of many hours, and neither is there any problem in visually distinguishing the material. Even if the body fluids coagulate during extended operations, because the surgical pad of the present invention is non-adhesive, there is no adhesion between the pad and the tissue of the patient so that when the surgical pad is taken away from the tissue no contusions occur.

In addition to being used as a cushioning material that protects veins and other types of delicate tissue, the surgical pad of the present invention can be used as a separation material used to ensure a boundary surface between normal tissue and a tumor after surgery to remove the tumor, among other applications. In such an application, the surgical pad will prevent tissue adhesions from occurring during surgery.

Referring to Fig. 1, a surgical pad 1 is provided with an optional pulling member or drawstring 2 which is useful for pulling the pad. The optional drawstring 2 is sinusoidally arranged as at 3 in order to facilitate attachment to the pad 1.

The shape of the pad 1 is discretionary. In addition to a rectangular shape, a shape can be adopted that is suited to the location in which the pad is to be used.

The optional drawstring or pulling member 2 can be in the form of a thread or a strip. If the member is to be in the form of a thread, any surgical suturing thread that is commonly used can be employed. If it is to be in the form of a strip, a porous expanded fluorine resin sheet, a fluorine resin sheet, or another polymer sheet that has been cut into a thin strip can be employed. This optional pulling member is attached to the pad in a suitable location and by any suitable means, such as thermal fusion or an adhesive agent.

After a pad having the optional pulling member has been used, its removal by use of the pulling member can be performed with the utmost of ease and safety. The embodiment incorporating the pulling member is therefore felt to represent the best mode of the present invention.

EXAMPLE 1

300 ml of naphtha was admixed as an extrusion aide with 1 kg of unsintered fine powder PTFE resin. After keeping this mixture at 40°C for 24 hours and thoroughly blending the naphtha in the resin, the product was pressed together and then extruded with a ram extruder and subjected to pressure rolling between a pair of metal rollers. The rolling treatment was repeated until a long sheet with a thickness of 150 $\mu$m was obtained.

After then drying this long sheet at 200°C to remove the naphtha, the sheet was stretched 300% in the lengthwise direction and 200% in the short axis direction to form a porous expanded PTFE sheet with a thickness of 120 $\mu$m and a porosity of 85%. The rate of stretch was 10 percent per second. Eight of these sheets were laminated together such that the fibrillar orientation of each sheet would be on the bias with its adjacent sheets. This laminate was sandwiched between two polished sheets of stainless steel, and this product was then subjected to heat and pressure bonding for 20 minutes at a pressure of 0.2 kg/cm$^2$ with a hot plate press that had been heated to 360°C. After this, the laminate was cooled to room temperature to produce Sample A, a porous expanded PTFE sheet with a multi-layer structure, a thickness of 800 $\mu$m (0.8 mm), and a porosity of 75%.

This Sample A was then further stretched 200% in both the lengthwise direction and the short axis direction, subjected to a heat treatment for 30 minutes at 370°C, and cooled, which resulted in Sample B being obtained. Sample B had a thickness of 700 $\mu$m (0.7 mm) and a porosity of 90%.

Upon using an electron microscope to observe the above Samples A and B, it was confirmed that the cross sections thereof had a uniform structure and that the eight laminated sheets had become completely integrated.

EXAMPLE 2

400 ml of mineral spirits were added as a working aid to 1 kg unsintered fine powder PTFE resin. After thorough mixing, the mixture was kept at 40°C for 24 hours while the resin and mineral spirits were thoroughly blended together. This mixture was subjected to repeated pressure rolling between metal rollers and worked into a sheet form. After then drying this sheet at 200°C to remove the mineral spirits, it was stretched 300% in only the lengthwise direction, then subjected to a heat treatment for 30 minutes at 380°C to produce a Sample C. Sample C had a thickness of 250 $\mu$m and a porosity of 80%.

EXAMPLE 3

The porous expanded PTFE sheet produced in Example 1, which had a thickness of 120 $\mu$m and a porosity of 85%, was coated at spaced locations with a polyurethane-based adhesive agent in a coating amount of 10 g/m$^2$. Four of these coated sheets were laid on top of each other and then subjected to a heat treatment for one hour at 150°C to produce a laminate, Sample D, with a thickness of 500 $\mu$m.

EXAMPLE 4

Ten of the porous expanded PTFE sheets produced in Example 1, which had a thickness of 120 $\mu$m and a porosity of 85%, were laminated such that the fibrillar orientation of each of the sheets would be on the bias with its adjacent sheets. These sheets were sandwiched between sheets of stainless steel and the stainless steel sheets were subjected to a heat treatment with a hot plate press for 20 minutes at a temperature of 360°C and a pressure of 10 kg/cm$^2$. This product was cooled to room temperature to obtain Sample E. This Sample E had a thickness of 500 um and a porosity of 70%.

The shape conformability, edge flexibility, ease of visual distinction, shape and retention of the above Samples A to E from Examples 1 to 4 and of a commercially available hydrophilic cotton-based surgical pad and collagen-based surgical pad were then comparitively studied according to the following descriptions. The results of these comparisons are shown in Table 1.

Shape Conformability

A tester with a total weight of approximately 200 g was prepared by winding a stainless steel wire with a diameter of 1 mm around a stainless steel rod having an external diameter of 12 mm so that there were no gaps between the windings.

Separately, several samples cut to a size of 20 x 50 mm were laid over a smooth glass sheet with a thickness of approximately 7 mm such that the thickness of the sheets would be approximately 1 mm. The above-mentioned tester was gently placed on these overlaid samples in roughly the center, and was left for five minutes at room temperature. After the five minutes had elapsed, the tester was gently removed from the samples, and the surface of the samples was observed. The uppermost sample was then removed, the edges of the sample held, the sample was slowly and gently stretched out, and the state of the surface thereof was observed and evaluated with the following standards.

Excellent: The shape of the tester remained distinctly on the surface, and when the surface was stretched out, the shape of the tester disappeared completely.

Good: The shape of the tester remained for the most part on the surface, and when the surface was stretched out, the shape of the tester virtually disappeared.

Poor: The shape of the tester remained only very faintly, or was absent.

Edge Flexibility

Commercially available, fresh soft tofu that had been cut into squares of 50 mm x 50 mm x 30 mm was placed on a smooth glass sheet.

Meanwhile, the sample sheets were cut into 10 mm x 50 mm sizes. The cut samples were carefully sandwiched with no creases formed between flat plastic sheets 20 mm wide, 100 mm long, and 1.5 mm thick such that the end of the samples would protrude approximately 5 mm. This protruding end was pressed gently and perpendicularly against the surface of the previously mentioned tofu. Those samples in which the end curled up and would not penetrate the tofu were deemed good and those in which the end would not curl up, but penetrated into the tofu, were deemed poor.

Ease of Visual Distinction

The samples were cut into 10 mm x 50 mm sizes and put into a laboratory dish with a diameter of 90 mm and which had been filed with fresh cow or pig blood, and this dish was covered with a lid and let stand for approximately one hour. The samples were lifted out of the lids, and those which had absorbed or adsorbed the blood and been dyed by the blood were deemed poor, while those which had absorbed or adsorbed hardly any blood and were not dyed were deemed good.

Shape Retention

The shape of the samples was observed after they had been observed for ease of visual distinction. Those in which the post-test shape had changed hardly at all from the pre-test shape were deemed good, and those in which there was considerable change were deemed poor.

TABLE 1

| Cushioning Material | Thickness (μm) | Percentage of Void (%) | Shape Conformability | Edge Flexibility | Ease Of Visual Distinction | Shape Retention |
|---|---|---|---|---|---|---|
| Sample A | 800 | 75 | good | good | good | good |
| Sample B | 700 | 90 | excellent | good | good | good |
| Sample C | 250 | 80 | good | good | good | good |
| Sample D | 500 | 85 | good | good | good | good |
| Sample E | 500 | 70 | poor | poor | poor | good |
| Commercially Available Cotton Non-Woven Cloth | 1000 | - | poor resilient | good | poor | poor swelled |
| Commercially Available Collagen Sheet | 450 | - | poor resilient | good | poor | poor swelled |

As can be seen from the results in Table 1, the products of the present invention (Samples A through D) were vastly superior to the conventional products in terms of being used as surgical pads for use in surgical operations.

**Claims**

1.  A surgical pad comprising an expanded, porous fluorine resin sheet having a porosity of 75 to 95% and a thickness of at least about 0.1 mm.

2.  A surgical pad according to claim 1 wherein the pad has a thickness of less than about 1.0 mm.

3.  A surgical pad according to claim 1 or 2, comprising a laminate composed of a plurality of the fluorine resin sheets.

4.  A surgical pad according to any of claims 1 to 3, wherein the fluorine resin is polytetrafluoroethylene.

5.  A surgical pad according to any preceding claim, wherein a pulling member is attached to the surgical pad.

6.  A method of padding living tissue during surgery comprising padding the living tissue with a surgical pad as claimed in any preceding claim.

7.  A method of preventing tissue adhesions from occurring between adjacent tissues during surgery, comprising placing a surgical pad between the adjacent tissues, wherein the surgical pad is as claimed in any of claims 1 to 5.

European Patent
Office

PARTIAL EUROPEAN SEARCH REPORT

which under Rule 45 of the European Patent Convention
shall be considered, for the purposes of subsequent
proceedings, as the European search report

Application Number

EP    91 30 7097

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 542 605  (J. CINQUALBRE)  * Abstract; page 1, lines 6-16; page 3, line 34 - page 4, line 4 * | 1-2,4-5 | A 61 L   31/00  A 61 F   13/36 |
| Y |  | 3 |  |
| D,Y | US-A-4 187 390  (W.L. GORE & ASSOCIATES)  * Abstract; column 4, lines 36-39; column 5, lines 44-46; column 8, lines 19-29; column 15, lines 29-32; claim 35 * | 3 |  |
| A |  | 1,4 |  |
| A | WO-A-9 014 810  (R. BERGUER et al.)  * Page 8, lines 5-17 * | 1-2 |  |
| A | FR-A-2 281 448  (IMPERIAL CHEMICAL INDUSTRIES)  * Page 14, lines 1-8,21-28; page 15, lines 13-14 *                       -/- | 1-2 |  |

TECHNICAL FIELDS
SEARCHED (Int. Cl.5)

A 61 F
A 61 L

## INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :     1-5
Claims searched incompletely :
Claims not searched :     6-7
Reason for the limitation of the search:

Method for treatment of the human or
animal body by surgery or therapy
(see article 52(4) of the European
Patent Convention)

| Place of search | Date of completion of the search | Examiner |
|---|---|---|
| THE HAGUE | 30-03-1992 | NICE P.R. |

European Patent
Office

**PARTIAL EUROPEAN SEARCH REPORT**

Application Number

EP 91 30 7097

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| A | EP-A-0 277 009 (ACCU-MED) <br> * Abstract; figure 1 * | 1,5 | |
| A | EP-A-0 006 406 (FUJI SYSTEMS) <br> * Abstract; page 3, lines 21-23; figures 3-5 * | 1,5 | |

TECHNICAL FIELDS SEARCHED (Int. Cl.5)

EPO FORM 1503 03.82 (P0410)